# EUROPÄISCHE PATENTSCHRIFT

(11) **EP 3 745 131 B1**
(45) Veröffentlichungstag und Bekanntmachung des Hinweises auf die Patenterteilung: **30.03.2022**
(21) Anmeldenummer: 19177424.9
(22) Anmeldetag: 29.05.2019
(51) Int. Cl.: G01N 33/558, G01N 33/543

(54) **VERFAHREN UND MITTEL ZUR VERLÄNGERUNG DER AUSWERTZEIT EINES TESTSTREIFENS ZUM VISUELLEN NACHWEIS VON ANALYTEN**
METHOD AND MEANS FOR EXTENDING THE EVALUATION PERIOD OF A TEST STRIP FOR VISUAL DETECTION OF ANALYTES
PROCÉDÉ ET MOYEN DE PROLONGATION DU TEMPS D'ÉVALUATION D'UNE BANDE D'ESSAI DESTINÉE À LA DÉTECTION VISUELLE DES ANALYTES

(43) Veröffentlichungstag der Anmeldung: 02.12.2020
(73) Patentinhaber: Protzek Gesellschaft für Biomedizinische Technik GmbH, 4415 Lausen (CH)
(72) Erfinder: Protzek, Christoph, 79539 Lörrach (DE); Maba, Dao L., 79540 Lörrach (DE)
(74) Vertreter: Patentanwälte Dörner & Kötter PartG mbB

(56) Entgegenhaltungen:
- KR-A- 20180 121 168
- US-A1- 2002 028 438
- US-A1- 2012 156 693

## Beschreibung

Die Erfindung betrifft ein Verfahren zur Verlängerung der repräsentativen Auswertezeit eines mit einer vom menschlichen oder tierischen Organismus stammenden Probe beaufschlagten Teststreifens nach dem Oberbegriff des Patentanspruchs 1. Die Erfindung betrifft weiterhin ein Fixiermittel zur Verlängerung der repräsentativen Auswertezeit eines mit einer vom menschlichen oder tierischen Organismus stammenden Probe beaufschlagten Teststreifens nach dem Oberbegriff des Patentanspruchs 6. Die Erfindung betrifft darüber hinaus ein Set zur Prüfung und Anzeige der Ab- bzw. Anwesenheit von Analyten in einer vom menschlichen oder tierischen Organismus stammenden Flüssigkeitsprobe nach dem Patentanspruch 8.

Vorrichtungen zum Nachweis von Analyten in einer flüssigen Probe, zum Beispiel von Drogen, Medikamenten oder Antikörpern als Zeichen für bestimmte Erkrankungen in einer Körperflüssigkeit oder Feststellungen im Sinne von Konzentrationsangaben sind in unterschiedlichen Ausführungen bekannt und beispielsweise in der US 2012/156693 A1 beschrieben. Besonderes Interesse besteht an solchen Vorrichtungen, die auch außerhalb eines Labors eingesetzt werden können. Diese müssen tragbar und leicht zu bedienen sein sowie in kurzer Zeit ein zuverlässiges Ergebnis liefern. Insbesondere zur Durchführung von Tests auf Drogenkonsum oder Medikamentenmissbrauch oder dergleichen durch die Polizei ist eine Vorrichtung erforderlich, die auch im Freien bei unterschiedlichen wechselnden Lichtverhältnissen, Wetterverhältnissen und Temperaturen einen derartigen Test ermöglicht. In der DE 20 2014 002 369 U1 wird hierzu ein Gerät vorgeschlagen, in das eine Testkassette einführbar ist und das mit einer Lichtquelle versehen ist, mittels derer die untere Wand der aus einem lichtdurchlässigem Kunststoff bestehenden Testkassette sowie der Testbereich des in der Testkassette angeordneten Teststreifens durchstrahlt und das Testergebnis ausgeleuchtet werden kann. Die Testkassette ist zur Durchführung eines lateral flow immuno-assays, Aptamer basierter Assay oder Encym immuno assay (EIA) ausgelegt und besteht aus einem unteren Deckel und einem oberen Deckel. Eingeschlossen von diesen beiden Deckeln enthält die Testkassette einen Teststreifen, der innerhalb einer Aufnahme in Position gehalten wird. In dem oberen Deckel befindet sich über dem Probenaufnehmer des Teststreifens eine Einfüllöffnung, durch die eine zu untersuchende flüssige Probe eingegeben werden kann. Über dem mittleren Testbereich des Teststreifens befindet sich ein Kontrollfenster, durch welches das Testergebnis abgelesen werden kann. Derartige Kassetten haben sich im mobilen Einsatz bewährt.

In der DE 20 2016 104 645 U1 ist eine kompakte, leichte und umweltfreundlich zu entsorgende Weiterentwicklung einer solchen Testkassette beschrieben. Hierbei ist eine Trägerplatte vorgesehen, die aus Kartonage bzw. Pappe gebildet ist. Die Teststreifen sind in Teststreifenaufnahmen eingelegt, die in Form von Einprägungen oder Ausnehmungen gebildet sind. Die so gestaltete Trägerplatte ist mit einer bereichsweise transparenten, verrottbaren Deckfolie versehen, die zur Probeaufgabe auf die Teststreifen mit einem Fenster versehen ist. Die Trägerplatte ist mit einem Deckel versehen, der einstückig an die Trägerplatte angeformt ist und der ebenfalls mit Teststreifen bestückte Teststreifenaufnahmen aufweisen kann.

In den vorbekannten Vorrichtungen werden sogenannte Immuno-assay-Teststreifen eingesetzt. Diese weisen an einem Ende einen Probeaufnahmeabschnitt und an ihrem anderen Ende eine Absorberschicht auf, zwischen denen sich ein Testabschnitt befindet, der mit der oder den Reagenzien für den oder die zu ermittelnden Analyten behandelt oder imprägniert ist. Vor dem Testabschnitt ist ein Antikörperbelag angeordnet, der Antikörper enthält, die mit Goldpartikeln konjugiert sind und die zu einem festzustellenden Analyten bzw. zu einem Antigen dieses Analyten passen und in eine immunologische Reaktion treten können. Der Testabschnitt ist in Form einer Membran ausgebildet, auf der sich beabstandet zueinander eine oder mehrere Testlinien und eine Kontrolllinie befinden, die durch einen Reagenz für den Nachweis jeweils eines in einem bestimmten Analyten enthaltenen Antigen ausgebildet und vor Aufgabe einer Probe unsichtbar sind.

Bei der Durchführung eines Tests wird eine Probeflüssigkeit auf den Probenaufnahmeabschnitt des Teststreifens aufgegeben, die durch Kapillarwirkung in Richtung Testabschnitt fließt, wobei sie zunächst mit dem Antikörperbelag in Kontakt kommt, in dem sich mit Goldpartikeln konjugierte Antikörper zum Antigen eines festzustellenden Analyten befinden. Die Antikörper weisen eine für verschiedene Analyten unterschiedliche Bindungsaktivität auf. Die mit Goldpartikeln konjugierten Antikörper werden durch die Probenflüssigkeit mitgenommen, welche den Testabschnitt durchfließt. Enthält die Probenflüssigkeit nicht den festzustellenden Analyten, so werden beim Durchfließen des Testabschnitts die dort im Bereich der jeweiligen Testlinie befindlichen Antigene des Analyten durch die von der Probenflüssigkeit mitgenommenen Antikörper gebunden und es bildet sich eine sichtbare Testlinie heraus, die durch die mit den Antikörpern konjugierten Goldpartikel eine rote oder braune Farbe erhält. Enthält die Probenflüssigkeit den festzustellenden Analyten, so werden dessen Antigene bereits durch die Antikörper im Bereich des Antiköperbelags gebunden und es kann im Testabschnitt nicht mehr zu einer Anlagerung kommen, weshalb sich keine sichtbare Testlinie herausbildet. Die Kontrolllinie gibt an ob eine ausreichende Probemenge aufgegeben worden ist, also ob der Test valide ist und seine Durchführung sachgemäß erfolgte.

Bei der Auswertung einer immunologischen Analyse im Lateral Flow Design ist die Intensität der Testlinien und ihre Stabilität zum Ende bzw. auch nach Ablauf der Testreaktion ein wichtiger und charakteristischer Faktor. Dieser ist unter anderen Faktoren von der Inkubationszeit abhängig, die folglich einen Schlüsselfaktor immunologischer Analysen darstellt. Sowohl bei der visuellen, als auch bei der opto-elektronischen Analyse kann sich das Testergebnis nach der vorgegeben Ablesezeit verändern.

Bei der visuellen, wie bei der opto-elektronischen Ergebnisgewinnung muss daher die Ablesezeit der Ergebnisse für jeden Test in einen Zusammenhang mit der benötigten Inkubationszeit gestellt werden. Nachteilig an den vorbekannten Arten oder Vorrichtungen zur Immunoassay-Analyse ist, dass die Auswertung in der Regel unmittelbar nach einem begrenzten Auswertungszeitraum (repräsentative Auswertezeit) bzw. unmittelbar im Anschluss an eine vorgegebene Reaktionszeit erfolgen muss. Danach ist mit einer Veränderung oder auch Verfälschung des ursprünglichen Unter-suchungs-ergebnisses zu rechnen, z.B. wegen nachträglicher Einstellung oder Veränderungen des Gleichgewichts von Reaktionspartnern oder wegen reaktiver Umwelteinflüsse auf die auf dem Testabschnitt befindliche Probe. Diese sollen möglichst ausgeschlossen werden. Beispielsweise führen (Verkehrs-) Polizisten in der Regel Ermittlungen außerhalb ihrer Geschäftsräume aus - oft unter zweifelhaften Bedingungen. Im Nachhinein kann es dazu kommen, dass ein unter diesen Bedingungen erhaltenes Testergebnis in einem Beweisverfahren angezweifelt oder gar ausgeschlossen wird, da eine nachträgliche Analyse zur Bestätigung dieses Testergebnisses unter Laborbedingungen nicht möglich ist.

Hier will die Erfindung Abhilfe schaffen. Der Erfindung liegt die Aufgabe zugrunde, ein Verfahren bereitzustellen, das eine Verlängerung der repräsentativen Auswertezeit eines mit einer vom menschlichen oder tierischen Körper stammenden Probe beaufschlagten Immuno-assay-Teststreifens ermöglicht. Gemäß der Erfindung wird diese Aufgabe durch ein Verfahren mit den Merkmalen des kennzeichnenden Teils des Patentanspruchs 1 gelöst.

Mit der Erfindung ist ein Verfahren bereitgestellt, dass eine Verlängerung der repräsentativen Auswertezeit eines mit einer vom menschlichen oder tierischen Körper stammenden Probe beaufschlagten Immuno-assay-Teststreifens ermöglicht. Dadurch, dass nach Ablauf einer spezifischen Inkubationszeit auf den Teststreifen ein fluides, transparentes und schnell trocknendes Fixiermittel aufgegeben wird, ist einer Beeinträchtigung der auf dem Testabschnitt durch äußere reaktive Einflüsse für einen weiteren Zeitraum entgegengewirkt. Im Falle einer schnellen Analyse im mobilen Einsatz kann die analysierte Probe durch die Aufgabe des Fixiermittels für eine spätere Auswertung konserviert werden. Das Fixiermittel ist auf Silikonbasis gebildet. Das Fixierungsmittel kann mit einer Pipette auf den Testabschnitt des Teststreifens aufgebracht werden.

In Ausgestaltung der Erfindung wird der Teststreifen auf einer Trägerkarte angeordnet und die Aufgabe des Fixiermittels erfolgt durch Einschieben der Trägerkarte in eine einen Einschub aufweisende Aufnahmevorrichtung, die ein mit Fixiermittel befülltes Reservoir umfasst, das mit einer Dosiereinrichtung verbunden ist, die zum Aufbringen des Fixiermittels auf den Teststreifen betätigt wird. Hierdurch ist die Aufgabe einer definierten Menge an Fixiermittel auf den einen definierten Abschnitt des Teststreifens ermöglicht. Bevorzugt wird die Dosiereinrichtung durch den Einschiebevorgang betätigt.

In weiterer Ausgestaltung der Erfindung ist die Aufnahmevorrichtung als Gerät zur visuellen Auswertung des Teststreifens ausgebildet, das bevorzugt Mittel zur Ausleuchtung des Teststreifens umfasst. Hierdurch ist eine schnelle Auswertung eines Teststreifens vor Ort mit nachfolgender Fixierung des Testabschnitts zur Verlängerung der repräsentativen Auswertezeit ermöglicht.

Der Erfindung liegt weiterhin die Aufgabe zugrunde, ein Fixiermittel zur Verwendung in einem solchen Verfahren bereitzustellen, das eine Verlängerung der repräsentativen Auswertungszeit eines mit einer vom menschlichen oder tierischen Körper stammenden Probe beaufschlagten Immuno-assay-Teststreifens ermöglicht. Gemäß der Erfindung wird diese Aufgabe durch ein Fixiermittel gelöst, das im Wesentlichen aus Cyclohexasiloxan, Dimethicon und Disiloxan besteht, wobei wenigstens 55% Cyclohexasiloxan und wenigstens 28% Dimethicon und wenigstens 3% Disiloxan enthalten sind.

In Weiterbildung der Erfindung sind wenigstens 60%, bevorzugt 62% Cyclohexasiloxan sowie wenigstens 30%, bevorzugt 32% Dimethicon enthalten. Dabei sind besonders bevorzugt 5% Disiloxan enthalten. Es hat sich gezeigt, dass hierdurch eine sehr schnelle Trocknung des Fixiermittels sowie eine signifikante Erhöhung der Auswertezeit erzielbar sind.

Der Erfindung liegt darüber hinaus die Aufgabe zugrunde, ein Set zur Durchführung des vorgenannten Verfahrens bereitzustellen, das eine Verlängerung der repräsentativen Auswertungszeit eines mit einer vom menschlichen oder tierisehen Körper stammenden Probe beaufschlagten Immuno-assay-Teststreifens ermöglicht. Gemäß der Erfindung wird diese Aufgabe durch ein Set mit den Merkmalen des Patentanspruchs 8 gelöst. Dadurch, dass eine Trägerplatte mit wenigstens einer Teststreifenaufnahme vorhanden ist, in der ein mit einem Probeaufnahmeabschnitt und einem Testabschnitt versehener Teststreifen angeordnet ist, der mit auf die diese Analyten abgestimmten, mit Farbpartikeln, insbesondere Goldpartikeln konjugierten Antikörpern versehen ist, wobei wenigstens ein mit einem Fixierungsmittel befülltes Reservoir angeordnet ist, das mit einer Dosiereinrichtung zur Aufbringung des Fixierungsmittels auf den Teststreifen verbunden ist, ist eine mobile Durchführung einer Immuno-assay-Analyse nach Aufgabe einer flüssigen Probe mit visueller oder opto-elektronischen Auswertung vor Ort mit anschließender Konservierung der ausgewerteten Probe auf dem Testabschnitt des Teststreifens ermöglicht, wodurch eine Erhöhung der Auswertezeit bewirkt ist, wodurch wiederum eine Wiederholung der Auswertung unter Laborbedingungen zu einem späteren Zeitpunkt ermöglicht ist.

In Weiterbildung der Erfindung ist das Reservoir in Form einer Pipette, insbesondere einer Einwegpipette ausgebildet. Diese Pipette kann dem Set separat beigefügt oder auch über eine Solltrennverbindung mit der Trägerplatte verbunden sein. Vorzugsweise weist die Solltrennverbindung eine Perforationslinie auf.

In Ausgestaltung der Erfindung umfasst das Set ein Gerät zur visuellen Auswertung des Testabschnitts wenigstens eines auf der Trägerplatte angeordneten Teststreifens, das einen Einschub zur Aufnahme der Trägerplatte sowie ein mit einem Fixiermittel befülltes Reservoir aufweist, das mit einer Dosiereinrichtung verbunden ist, über die Fixiermittel aus dem Reservoir auf den Probeaufgabenabschnitt des wenigstens einen Teststreifens der eingeschobenen Trägerkarte aufgebbar ist. Hierdurch ist eine mobile Durchführung einer Immuno-assay-Analyse nach Aufgabe einer flüssigen Probe mit opto-elektronischer Auswertung vor Ort mit anschließender Konservierung der ausgewerteten Probe auf dem Testabschnitt des Teststreifens ermöglicht.

In alternativer Ausgestaltung der Erfindung ist eine Hülle zur abdeckenden Aufnahme der Trägerplatte vorhanden, an der das Reservoir derart angebracht ist, des es nach bestimmungsgemäßer Aufnahme der Trägerplatte über dem Probeaufgabeabschnitt des wenigstens einen auf der Trägerplatte angeordneten Teststreifens angeordnet ist. Bevorzugt sind auf der Trägerplatte Mittel zur Öffnung des Reservoirs vorhanden, die derart positioniert sind, dass sie beim Einschieben der Trägerplatte in die Hülle das Reservoir derart passieren, dass dieses geöffnet wird. Dabei sind die Mittel zur Öffnung des Reservoirs bevorzugt in Form eines Schneid- oder Trennelements ausgebildet. Nach Öffnung des Reservoirs ist so eine definierte Beaufschlagung des Probeaufgabeabschnitts mit Fixierungsmittel gewährleistet.

In Weiterbildung der Erfindung ist das Reservoir durch eine Folienfalte gebildet, deren Öffnung über einen definierten Verschlussstreifen auf der Trägerplatte verschlossen ist, der über das Schneid- oder Trennelement lösbar oder trennbar ist. Hierdurch ist nach Öffnung des Reservoirs ein definierter Ausfluss vorhanden, wodurch eine definierte Beaufschlagung des Testabschnitts des auf der Trägerplatte positionierten Teststreifens ermöglicht ist.

Die Erfindung liegt weiterhin die Aufgabe zu Grunde, ein Gerät zur visuellen Auswertung wenigstens eines auf einer Trägerplatte angeordneten, einen Probeaufnahmeabschnitt und einen Testabschnitt aufweisenden Teststreifens eines kompetitiven Immuno-Assays zur Verwendung in einem Set der vorgenannten Art bereitzustellen, das eine Verlängerung der repräsentativen Auswertungszeit eines mit einer vom menschlichen oder tierischen Körper stammenden Probe beaufschlagten Teststreifens ermöglicht. Gemäß der Erfindung wird diese Aufgabe durch ein Gerät mit den Merkmalen des Patentanspruchs 12 gelöst.

Dadurch, dass ein mit einem Fixiermittel befülltes Reservoir angeordnet ist, das mit einer Dosiervorrichtung zur Aufbringung von Fixiermittel auf den Probeaufnahmeabschnitt wenigstens eines Teststreifens einer von dem Einschub aufgenommenen Trägerkarte verbunden ist, ist eine elektro-optische Auswertung eines auf der Trägerplatte angeordneten Teststreifens mit anschließender Fixierung ermöglicht, welche aufgrund der definierten Positionierung des Dosiermittels über dem Probeaufgabeabschnitt eines Teststreifens einer eingeschobenen Trägerplatte in definierter Art und Weise erfolgen kann, ohne dass es besondere Fertigkeiten des Bedieners erfordert.

Das Dosiermittel kann beispielsweise ein elektrisch betätigbares Ventil umfassen, dass über einen an dem Gerät angeordneten Bedienknopf steuerbar ist oder das automatisch nach einer definierten Verweilzeit - nach Ablauf der Inkubationszeit - über ein angeordnetes Steuergerät betätigbar ist. Die Dosiervorrichtung kann auch einen Kolben aufweisen, der über ein angeordnetes Steuergerät betätigbar ist und über den eine definierte Menge aus dem Reservoir ausbringbar ist.

In weiterer Ausgestaltung der Erfindung ist das Reservoir in Form einer Kartusche ausgebildet, die lösbar in einer Kartuschenaufnahme des Gerätes angeordnet ist. Hierdurch ist ein einfacher Wechsel einer leeren Kartusche gegen eine befüllte Kartusche ermöglicht. Bevorzugt ist die Kartusche mit einem Fixiermittel mit den Merkmalen eines der Patentansprüche 7 und 8 befüllt.

Gegenstand der vorliegenden Erfindung ist darüber hinaus auch eine Kartusche zum Einsatz in einem solchen Gerät, welche mit einem Fixiermittel befüllt ist, das im Wesentlichen aus Cyclohexasiloxan, Dimethicon und Disiloxan besteht, wobei wenigstens 55% Cyclohexasiloxan, wenigstens 28 % Dimethicon und wenigstens 3% Disiloxan enthalten sind.

Andere Weiterbildungen und Ausgestaltungen der Erfindung sind in den übrigen Unteransprüchen angegeben. Ein Ausführungsbeispiel der Erfindung ist in den Zeichnungen dargestellt und wird nachfolgend im Einzelnen beschrieben. Es zeigen:
- Figur 1: Die schematische Darstellung eines Gerätes zur visuellen Auswertung eines Immuno-assay-Teststreifens;
- Figur 2: die schematische Darstellung einer Trägerplatte mit zwei Teststreifen;
- Figur 3: die schematische Darstellung eines Teststreifens der Tägerplatte aus Figur 2 im Längsschnitt;

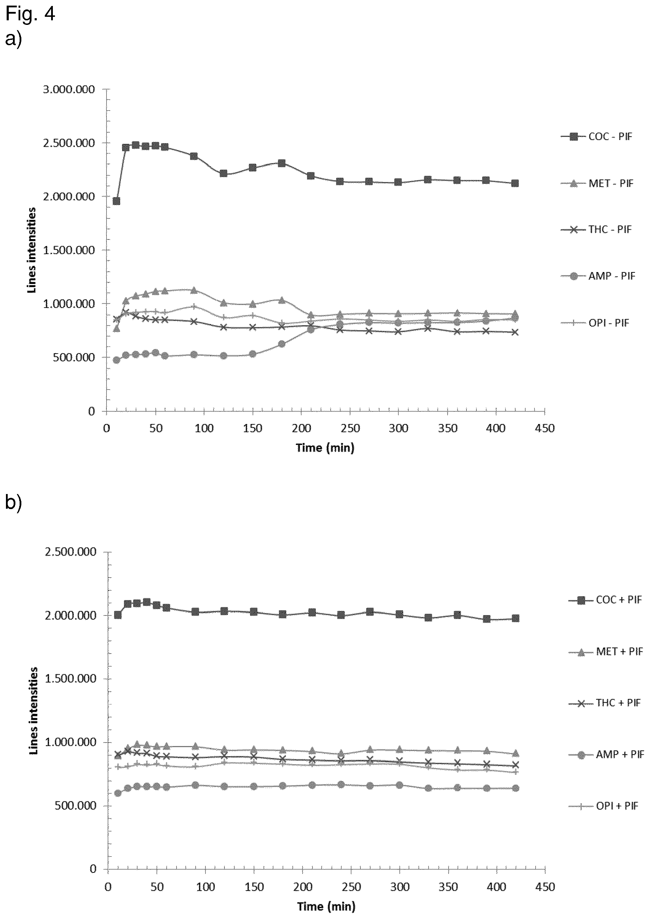

- Figur 4: die Darstellung der Ergebnisse einer Immuno-assay-Analyse unterschiedlicher Proben a) ohne Fixiermittel; b) mit Fixiermittel.

Das als Ausführungsbeispiel gewählte Gerät 1 zur visuellen Auswertung eines Immuno-assay-Teststreifens umfasst ein Gehäuse 11, in dem eine elektronische Platine 12 mit elektronischen Bauelementen und Microcontroller 121 sowie eine Lichtquelle in Form von drei LEDs 122, die ein standardisiertes Licht emittieren, angeordnet ist. Über den LEDs 122 ist in der Deckseite 13 des Gehäuses 11 ein transparentes Fenster 14 angeordnet, durch das Licht von den LEDs 46 hindurchtreten kann.

Auf der Deckseite 13 des Gehäuses 11 ist ein Einschub 15 zur Aufnahme einer mit Teststreifen 3 versehenen Trägerkarte 14 angeordnet. Auf dem Einschub 15 ist eine Kartuschenaufnahme 2 angeordnet, die mit einer Dosiereinrichtung 21 verbunden ist, die über einer in dem Einschub 15 eingebrachten Öffnung 16 positioniert ist. Die Kartuschenaufnahme 2 nimmt eine Kartusche 22 auf, die mit einem flüssigen Fixiermittel befüllt ist. Endseitig ist in dem Einschub 15 weiterhin eine Lichtschranke 17 zur Detektierung einer eingeschobenen Trägerkarte 4 angeordnet. Weiterhin sind auf der Deckseite 13 des Gehäuses 11 Bedientasten 18 zur Ansteuerung der LEDs 122 sowie der Dosiereinrichtung 21 angeordnet.

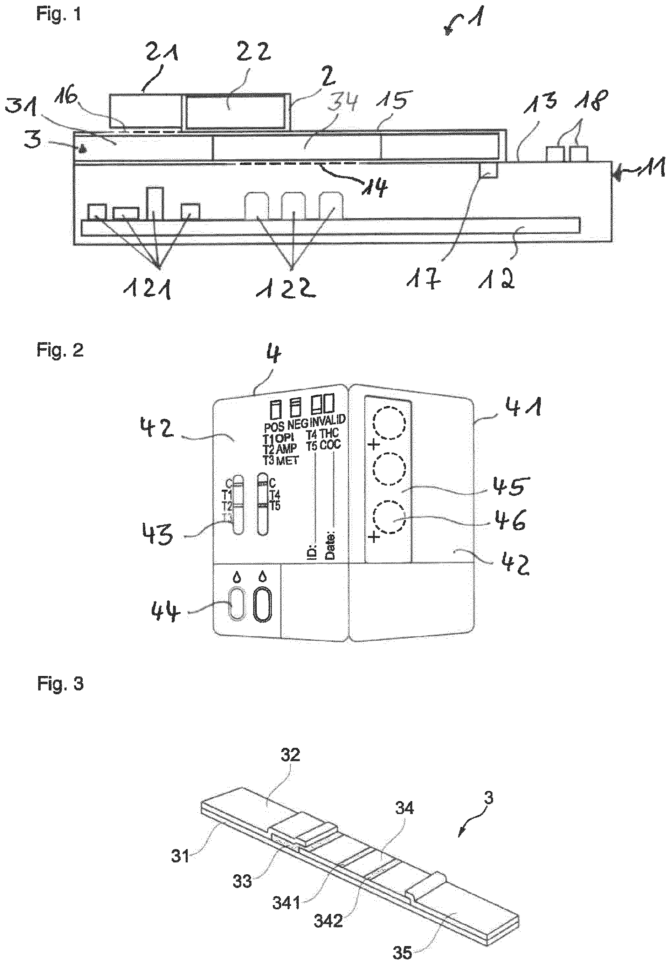

In Figur 2 ist eine Trägerplatte 4 zum Einschieben in den Einschub 15 des Gerätes 1 dargestellt. Eine solche Trägerplatte ist beispielsweise in der EP 3 361 253 A1 beschrieben. Die Trägerplatte 4 weist seitlich einen schwenkbar mit dieser verbundenen Deckel 41 auf. Im Ausführungsbeispiel sind Trägerplatte 4 und Deckel 41 aus einem einzigen, mittig mit einem Knickfalz versehen Kartonzuschnitt hergestellt. Auf die Trägerplatte 4 ist eine Zwischenschicht aufgebracht, die zwei Teststreifen 3 aufnimmt und die mit einer Deckschicht 42 versehen ist. Im Ausführungsbeispiel ist die Deckschicht 42 aus einer verrottbaren, farbigen Folie gebildet, die mit transparent ausgebildeten, klaren Kontrollfenstern 43 versehen ist.. Die Kontrollfenster 43 überspannen den jeweiligen Testabschnitt 34 der Teststreifen 3. Beabstandet zu den Kontrollfenstern 43 ist jeweils ein Probeaufgabefenster 44 angeordnet, das jeweils durch eine in der Deckschicht 42 eingebrachte Ausnehmung gebildet ist. Die Probeaufgabefenster 44 sind derart angeordnet, dass sie über den Probeaufnahmeabschnitten 32 der angeordneten Teststreifen 3 positioniert sind

Auf dem Deckel 41 ist im Ausführungsbeispiel ein DS-Probenträger 45 in Form einer Cellulosekarte angeordnet, welche mit drei kreisförmig markierten Probeaufgabebereichen 46 versehen ist. Auch der Deckel 41 ist mit einer Deckschicht 42 versehen, die im Bereich des DS-Probenträgers 45 eine Ausnehmung aufweist, die einen Zugang zu dem DS-Probenträger 45 ermöglicht.

Der Teststreifen 3 ist in Figur 3 im Stufenschnitt dargestellt. Er weist eine Trägerfolie 31 auf, auf der an einem Ende ein Probeaufnahmeabschnitt 32 angeordnet ist. Unmittelbar benachbart und teilweise unter dem Probeaufnahmeabschnitt 32 befindet sich auf der Trägerfolie 31 ein Antikörperbelag 33, der Antikörper enthält, die mit Goldpartikeln (oder auch mit anderen bzw. andersfarbigen Markerpartikeln) konjugiert sind und die zu einem festzustellenden Analyten bzw. zu einem Antigen dieses Analyten passen und in eine immunologische Reaktion treten können. An den Antikörperbelagabschnitt 33 schließt sich ein Testabschnitt 34 an, der in Form einer Membran ausgebildet ist, auf den sich beabstandet zueinander eine Testlinie 341 und eine Kontrolllinie 342 befinden, die durch in einem Reagenz für den Nachweis eines bestimmten Analyten enthaltenen Antigen ausgebildet sind und vor Aufgabe einer Probe unsichtbar sind. Der Probenaufnahmeabschnitt 32 des Teststreifens 3 befindet sich unterhalb der Probenaufgabefenster 44 und der Testabschnitt 34 befindet sich unterhalb des Kontrollfensters 43 der auf der Zwischenschicht der Trägerplatte 4 aufgebrachten Deckschicht 42.

Bei der Durchführung eines "lateral flow immuno-assays" zeigt die Testlinie 341 das Ergebnis des Tests an; die Kontrolllinie 342 gibt an, ob eine ausreichende Probemenge aufgegeben worden ist, also ob der Test valide ist. An seinem dem Probeaufnahmeabschnitt 32 gegenüberliegenden Ende ist an den Teststreifen 3 ein Absorberabschnitt 35 angeordnet.

Vor der Durchführung eines Tests sind keine Linien 341, 342 ersichtlich. Bei der Durchführung eines Tests wird ein Probevolumen einer Probeflüssigkeit durch ein Probeaufgabefenster 44 der Trägerplatte 4 auf den Probeaufnahmeabschnitt 32 eines jeden Teststreifens 3 aufgegeben. Durch Kapilarwirkung fließt die Probenflüssigkeit in Richtung Testabschnitt 34. Sie kommt dabei zunächst mit dem Antikörperbelagabschnitt 33 in Kontakt, in dem sich mit Goldpartikeln konjugierte Antikörper zum Antigen eines festzustellenden Analyten in standardisierter (dosierter) Menge befinden. Die Antikörper weisen eine für verschiedene Analyte (Drogen) unterschiedliche Bindungsaktivität auf. Die mit Goldpartikeln konjugierten Antikörper werden durch die Probenflüssigkeit mitgenommen, welche den Testabschnitt 34 durchfließt. Enthält die Probenflüssigkeit den festzustellenden Analyten nicht bzw. weniger als die im Test angelegte Anzeigegrenze, so binden beim Durchfließen des Testsabschnitts 34 die dort im Bereich der Testlinie 341 befindlichen Antigene des Analyten die von der Probenflüssigkeit mitgenommenen Antikörper. Es bildet sich daraufhin eine sichtbare Testlinie 341 heraus, die durch die mit den Antikörpern konjugierten Partikeln eine von den Partikeln abhängige Farbe erhält. Der Testbefund ist damit negativ.

Enthält die Probenflüssigkeit den festzustellenden Analyten, so wird diese als Antigen durch die Antikörper bereits im Bereich des Antikörperbelagabschnitts 33 des Teststreifens 3 gebunden und es kann im Testabschnitt 34 nicht mehr zu einer Anlagerung kommen, weshalb sich keine sichtbare Testlinie 341 herausbildet. Das Testergebnis ist in diesem Falle positiv. Die Kontrolllinie 342 zeigt an, ob der Test valide ist. In Figur 3 ist die Testlinie 341 farbig herausgebildet, weshalb der Test in Bezug auf den entsprechenden Analyten negativ ist. Zur visuellen Auswertung des Tests wird die Trägerplatte in den Einschub 15 des Gehäuses 11 des Analysegerätes 1 eingeschoben, wobei der Deckel 41 rückseitig umgeklappt und an die Trägerplatte 4 angelegt wird. Nach vollständiger Aufnahme der Trägerplatte 4 sind die Testabschnitte 34 der Teststreifen 3 über dem transparenten Fenster 14 der Deckseite 13 des Gehäuses 11 des Analysegerätes 1 angeordnet. Die Öffnung 16 der Deckseite 13 ist hierbei über dem Probeaufgabefenster 44 der Trägerplatte 4 positioniert. Durch Betätigung einer entsprechenden Bedientaste 18 werden die LEDs 122 der Platine 12 aktiviert, wodurch der Testabschnitt 34 der Teststreifen 3 beleuchtet wird. Im Ausführungsbeispiel werden für eine kontrastreiche Sichtbarmachung der Test- und Kontrolllinien Fluorophore eingesetzt, die auf den Immuno-assay-Teststreifen 3 aufgebracht werden. Diese Fluorophore lassen sich mit Ultraviolett-Licht anregen und strahlen dann ein sichtbares Licht in verschiedenen spektralen Bereichen abhängig vom Stoff aus; sie wirken selbst als eine Lichtquelle. Durch Dotierung der Fluorophore mit entsprechenden Aktivatoren lassen sich so verschiedene Farben erzeugen. Die Verwendung von Fluorophormolekülen auf den Immuno-assay-Teststreifen 3 sowie die zur Bestrahlung geeigneten Wellenlängen sind in der DE 20 2014 002 369 U1 beschrieben, die ein Gerät zur visuellen Auswertung eines Teststreifens zum Gegenstand hat.

Nach Abwarten der Inkubationszeit, die regelmäßig - je nach Analyt - etwa 10 Minuten beträgt (Standard-Inkubationszeit), wird durch Betätigung der entsprechenden Bedientaste 18 über die Dosiereinrichtung 21 in der Kartusche 22 befindliches flüssiges Fixiermittel durch die Öffnung 16 der Deckseite 13 des Gehäuses 11 durch die Probeaufgabefenster 44 der Trägerplatte 4 auf die Probeaufgabeabschnitte 32 der Teststreifen 3 aufgebracht. Die Fixierflüssigkeit gelangt durch Kapilarwirkung in den Testabschnitt 34 der Teststreifen, wo sie eintrocknet, wodurch eine Verlängerung der repräsentierten Auswertezeit der Teststreifen 3 bewirkt ist.

Im Ausführungsbeispiel ist die Fixierflüssigkeit aus 62% Cyclohexasiloxan, 33% Dimeticone sowie 5% Disiloxane gebildet.

Cyclohexasiloxane (D6) gehören zur Gruppe der Siloxane und sind organischen Silikonverbindungen. Gemische aus Cyclomethicon D5 und D6 sind frei auf dem Markt erhältlich und finden häufigen Gebrauch in der Kosmetik. Cyclomethicon hat die chemische Formel [Si(CH3)2O]ₙ, wobei n Werte zwischen 3 und 6 annehmen kann. Es ist eine flüchtige Dimethylpolysiloxan-Verbindung, welche die physikalische Gestalt einer Flüssigkeit mit niedriger Viskosität aufweist (in der Größenordnung von 2,5 mm²bis 6 mm² / s bei 25 Grad Celsius) und eine Oberflächenspannung von etwa 18-21 mN/m (ebenfalls bei 25 Grad Celsius) hat.

Dimethicon hat die chemische Formel CH3[Si(CH3)2O]Si(CH3)3. Dimethicon ist ein farbloses Silikonöl aus Dimethylsiloxan-Polymeren.

Disiloxan hat die chemische Formel Si₂H₆O. Disiloxan tritt unter Standartbedingungen als farbloses, ätzendes Gas auf. Disiloxane zeichnen sich dadurch aus, dass sie sehr schnell trocknen.

Das erfindungsgemäße Fixiermittel ist so konzipiert, dass es durch eine Pipette aufgetragen werden kann. Mit den schnelltrocknenden Tropfen können die erhaltenen Linienintensitäten des Testabschnitts der Teststreifen nach der Inkubationszeit für 4 Stunden und mehr Konserviert werden. Dabei enthält das erfindungsgemäße Fixiermittel keine Substanz, die sich nachteilig auf die Gesundheit oder auf die Umwelt auswirkt.

In Figur 3 sind Analyseergebnisse von Teststreifen, die mit unterschiedlichen Proben beaufschlagt wurden, dargestellt. Dabei zeigt Figur 3a die Ergebnisse der Teststreifens ohne Fixiermittel und Figur 3b die Analyseergebnisse nach Auftrag des Fixierungsmittels nach Ablauf der Standard-Inkubationszeit (10 Minuten). Folgende Proben wurden als 50% konzentrierte Lösungen auf Immunoassay-Teststreifen aufgegeben: OPI (Opiate), AMP (Amphetamine ("Speed"), MET (Methamphetamine), COC (Kokain), THC-COOH (Canabis).

Wie aus den Diagrammen unmittelbar ersichtlich ist, bewirkt das erfindungsgemäße Fixiermittel eine nahezu konstante Intensität der Testlinien über eine Zeit von mehr als 6 Stunden. Insbesondere die sich innerhalb der ersten 60 Minuten abzeichnenden deutlichen Veränderungen der Testlinien sind nach Auftrag des Fixiermittels nahezu nicht vorhanden. Das erfindungsgemäße Fixiermittel macht daher Auswertungen mit Messungen über einen langen Zeitraum möglich und erlaubt es zudem, dieselbe Probe für visuelle sowie auch im Nachgang für optoelektrische Analysen zu nutzen. Die vor Ort im Zuge eine Schnelltests durchgeführte Analyse kann unter Anwendung des erfindungsgemäßen Fixiermittels zuverlässig für eine spätere quantitative Analyse konserviert werden.

## Patentansprüche

1. Verfahren zur Verlängerung der repräsentativen Auswertezeit eines mit einer vom menschlichen oder tierischen Organismus stammenden Probe beaufschlagten Teststreifens (3) eines kompetitiven Immuno-assays, der zur Aufgabe der Probe einen Probeaufnahmeabschnitt (32) und zur Anzeige der An- bzw. Abwesenheit von definierten Analyten in der Probe einen Testabschnitt (34) aufweist, der mit auf diese abgestimmten Farbpartikeln, insbesondere auch Goldpartikeln konjugierten Antikörpern versehen ist, **dadurch gekennzeichnet, dass** nach Ablauf einer spezifischen Inkubationszeit auf dem Teststreifen (3) ein fluides, transparentes und schnelltrocknendes Fixiermittel zur Konservierung der Linienintensitäten des Teststreifens aufgegeben wird, das auf Silikonbasis gebildet ist.

2. Verfahren nach Anspruch 1, **dadurch gekennzeichnet, dass** das Fixiermittel auf den Probeaufnahmeabschnitt (32) aufgegeben wird.

3. Verfahren nach einem der vorgenannten Ansprüche, **dadurch gekennzeichnet, dass** der Teststreifen (3) auf einer Trägerkarte (4) angeordnet wird, wobei die Aufgabe des Fixiermittels durch Einschieben der Trägerkarte in eine einen Einschub (15) aufweisende Aufnahmevorrichtung erfolgt, die ein das Fixiermittel enthaltenes Reservoir (22) umfasst, das mit einer Dosiereinrichtung (21) verbunden ist, die zum Aufbringen des Fixiermittels auf den Teststeifen (3) betätigt wird.

4. Verfahren nach Anspruch 3, **dadurch gekennzeichnet, dass** die Dosiervorrichtung durch den Einschiebevorgang betätigt wird.

5. Verfahren nach Anspruch 3 oder 4, **dadurch gekennzeichnet, dass** die Aufnahmevorrichtung als Gerät zur visuellen Auswertung des Teststreifens ausgebildet ist.

6. Fixiermittel zur Verlängerung der repräsentativen Auswertezeit eines mit einer vom menschlichen oder tierischen Organismus stammenden Probe beaufschlagten Teststreifens (3) eines kompetitiven Immuno-assays zur Verwendung in einem Verfahren nach einem der vorgenannten Ansprüche, im Wesentlichen bestehend aus Cyclohexasiloxan, Dimethicon und Disiloxan, wobei wenigstens 55% Cyclohexasiloxan, wenigstens 28% Dimethicon und wenigstens 3% Disiloxan enthalten sind.

7. Fixiermittel nach Anspruch 6 **dadurch gekennzeichnet, dass** wenigstens 60%, bevorzugt 62% Cyclohexasiloxan, wenigstens 30%, bevorzugt 32% Dimethicon und wenigstens 4%, bevorzugt 5% Disiloxan enthalten sind.

8. Set zur Prüfung und Anzeige der Ab- bzw. Anwesenheit von Analyten in einer vom menschlichen oder tierischen Organismus stammenden Flüssigkeitsprobe, umfassend eine Trägerplatte (4) mit wenigstens einer Teststreifenaufnahme, in der wenigstens ein mit einem Probeaufnahmeabschnitt (32) und einem Testabschnitt (34) versehener Teststreifen (3) angeordnet ist, der mit auf diese Analyten abgestimmten, mit Farbpartikeln, insbesondere Goldpartikeln konjugierten Antikörpern versehen ist, **dadurch gekennzeichnet, dass** wenigstens ein mit einem zur Konservierung der Linienintensität des wenigstens einen Teststreifens (3) geeigneten Fixierungsmittel auf Silikonbasis befülltes Reservoir vorhanden ist, das mit einer Dosiereinrichtung zur Aufbringung des Fixierungsmittels auf den wenigstens einen Teststreifen verbunden ist.

9. Set nach Anspruch 8, **dadurch gekennzeichnet, dass** das Reservoir in Form einer Einwegpipette ausgebildet ist, die vorzugsweise über eine Solltrennverbindung mit der Trägerplatte (4) verbunden ist, die besonders bevorzugt eine Perforationslinie aufweist.

10. Set nach Anspruch 8, **dadurch gekennzeichnet, dass** eine Hülle zur abdeckenden Aufnahme der Trägerplatte (4) vorhanden ist, an der das Reservoir derart angebracht ist, dass es nach bestimmungsgemäßer Aufnahme der Trägerplatte (4) über dem Probeaufnahmeabschnitt (32) angeordnet ist, wobei bevorzugt auf der Trägerplatte (4) Mittel zur Öffnung des Reservoirs vorhanden sind, die derart positioniert sind, dass sie beim Einschieben der Trägerplatte (4) in die Hülle das Reservoir derart passieren, dass dieses geöffnet wird, wobei die Mittel zur Öffnung des Reservoirs bevorzugt in Form eines Schneid- oder Trennelements ausgebildet sind.

11. Set nach Anspruch 8, **dadurch gekennzeichnet, dass** dieses ein Gerät (1) zur visuellen Auswertung des Testabschnitts (34) des wenigstens einen Teststreifens (3) umfasst, das einen Einschub (15) zur Aufnahme der Trägerplatte (4) sowie ein mit einem Fixiermittel befülltes Reservoir (22) aufweist, das mit einer Dosiereinichtung (21) verbunden ist, über die Fixiermittel aus dem Reservoir (22) auf den Probeaufgabenabschnitt (32) des wenigstens einen Teststreifens (3) der eingeschobenen Trägerplatte (4) aufgebbar ist.

12. Gerät zur visuellen Auswertung wenigstens eines auf eine Trägerplatte (4) angeordneten, einen Probeaufnahmeabschnitt (32) und einen Testabschnitt (34) aufweisenden Teststreifens (3) eines kompetitiven Immuno-assays, mit einem Einschub (15) zur Aufnahme der Trägerplatte (4) sowie einer Beleuchtungseinheit zur gleichmäßigen Ausleuchtung der Trägerplatte (4), zumindest im Bereich des Testabschnitts (34) des wenigstens einen Teststreifens (3), **dadurch gekennzeichnet, dass** ein mit einem zur Konservierung der Linienintensität des wenigstens einen Teststreifens (3) geeigneten Fixiermittel auf Silikonbasis befülltes Reservoir (22) angeordnet ist, das mit einer Dosiereinrichtung (21) zur Aufbringung von Fixiermittel auf den Probeaufnahmeabschnitt (32) wenigstens eines Teststreifens (3) einer von dem Einschub (15) aufgenommenen Trägerplatte (4) verbunden ist.

13. Gerät nach Anspruch 12, **dadurch gekennzeichnet, dass** das Reservoir (22) als Kartusche ausgebildet ist, die lösbar in dem Gerät angeordnet ist.

14. Kartusche zur Verwendung in einem Gerät nach Anspruch 12 oder 13, **dadurch gekennzeichnet, dass** diese mit einem Fixiermittel nach Anspruch 6 oder 7 befüllt ist.

## Claims

1. Method for extending the representative evaluation period of a test strip (3) of a competitive immunoassay to which a sample derived from a human or animal organism has been applied, which strip has a sample receiving section (32) for applying a sample and a test section (34) for indicating the presence or absence of defined analytes in the sample, which is provided with antibodies conjugated with colour particles, in particular also gold particles, matched to these analytes, **characterised in that** after a specific incubation time has elapsed, a fluid, transparent and fast-drying fixative for preserving the line intensities of the test strip is applied to the test strip (3), which fixative is silicone-based.

2. Method according to claim 1, **characterised in that** the fixative is applied to the sample receiving section (32).

3. Method according to one of the previous claims, **characterised in that** the test strip (3) is arranged on a carrier card (4), wherein the fixative is applied by inserting the carrier card in a receiving device having a slot (15), which receiving device comprises a reservoir (22) containing the fixative, which is connected to a dosing device (21), which is operated to apply the fixative to the test strip (3).

4. Method according to claim 3, **characterised in that** the dosing device is actuated by the insertion process.

5. Method according to claim 3 or 4, **characterised in that** the receiving device is designed as a device for visual evaluation of the test strip.

6. Fixative for extending the representative evaluation period of a test strip (3) of a competitive immunoassay to which a sample derived from a human or animal organism has been applied for use in a method according to one of the previous claims, substantially consisting of cyclohexasiloxane, dimethicone and disiloxane, wherein at least 55% cyclohexasiloxane, at least 28% dimethicone and at least 3% disiloxane are contained.

7. Fixative according to claim 6, **characterised in that** at least 60%, preferably 62% cyclohexosiloxane, at least 30%, preferably 32% dimethicone and at least 4%, preferably 5% disiloxane are contained.

8. Set for testing and displaying the presence or absence of analytes in a liquid sample derived from the human or animal organism comprising a carrier plate (4) with at least one test strip receptacle, in which at least one test strip (3) provided with a sample receiving section (32) and a test section (34) is arranged, which is provided with antibodies matched to these analytes, conjugated with colour particles, in particular gold particles, **characterised in that** at least one reservoir filled with a silicone-based fixative suitable for preserving the line intensity of the at least one test strip (3) is provided, which reservoir is connected to a dosing device for applying the fixative to the at least one test strip.

9. Set according to claim 8, **characterised in that** the reservoir is designed in the form of a disposable pipette, which is preferably connected via a predetermined separation connection to the carrier plate (4), which particularly preferably has a perforation line.

10. Set according to claim 8, **characterised in that** a cover is provided for the covering accommodation of the carrier plate (4), on which the reservoir is mounted in such a manner that, after the carrier plate (4) has been received as intended, it is arranged above the sample receiving section (32), wherein means for opening the reservoir are preferably provided on the carrier plate (4), which are positioned in such a manner that, when the carrier plate (4) is inserted in the cover, they pass the reservoir in such a manner that it is opened, wherein the means for opening the reservoir are preferably designed in the form of a cutting or separating element.

11. Set according to claim 8, **characterised in that** it comprises a device (1) for visual evaluation of the test section (34) of the at least one test strip (3), which has a slot (15) for receiving the carrier plate (4) as well as a reservoir (22) filled with a fixative, which is connected to a dosing device (21), via which the fixative can be applied from the reservoir (22) to the sample application section (32) of the at least one test strip (3) of the inserted carrier plate (4).

12. Device for the visual evaluation of at least one test strip (3) of a competitive immunoassay arranged on a carrier plate (4) and having a sample receiving section (32) and a test section (34), with a slot (15) for receiving the carrier plate (4) as well as a lighting unit for the uniform illumination of the carrier plate (4), at least in the area of the test section (34) of the at least one test strip (3), **characterised in that** a reservoir (22) filled with a silicone-based fixative suitable for preserving the line intensity of the at least one test strip (3) is arranged, which is connected to a dosing device (21) for applying fixative to the sample receiving section (32) of at least one test strip (3) of a carrier plate (4) received by the slot (15).

13. Device according to claim 12, **characterised in that** the reservoir (22) is designed as a cartridge, which is detachably arranged in the device.

14. Cartridge for use in a device according to claim 12 or 13, **characterised in that** it is filled with a fixative according to claim 6 or 7.

## Revendications

1. Procédé pour prolonger le temps d'évaluation représentatif d'une bandelette réactive (3), d'un immunodosage par compétition, garnie d'un échantillon provenant de l'organisme humain ou animal, bandelette qui présente un segment réceptacle (32) d'échantillon où appliquer l'échantillon et un segment réactif (34) où afficher la présence ou l'absence d'analytes définis dans l'échantillon, bandelette qui est dotée d'anticorps harmonisés avec ces analytes et conjugués à des particules de couleur, notamment des particules d'or, **caractérisé en ce qu'**après l'expiration d'un temps d'incubation spécifique est versé sur la bandelette réactive (3) un fixateur fluide transparent et séchant rapidement, formulé à base de silicone, pour conserver les intensités des lignes de la bandelette réactive,

2. Procédé selon la revendication 1, **caractérisé en ce que** le fixateur est appliqué sur le segment réceptacle (32) de l'échantillon.

3. Procédé selon l'une des revendications précédentes, **caractérisé en ce que** la bandelette réactive (3) est disposée sur une carte support (4), sachant que l'application du fixateur a lieu en rentrant la carte support dans un dispositif réceptacle présentant un tiroir (15), dispositif qui comprend un réservoir (22) contenant le fixateur, réservoir qui est relié à un dispositif de dosage (21) actionné pour appliquer le fixateur sur la bande réactive (3).

4. Procédé selon la revendication 3, **caractérisé en ce que** le dispositif de dosage est actionné par l'opération de rentrée.

5. Procédé selon la revendication 3 ou 4, **caractérisé en ce que** le dispositif réceptacle est configuré comme un appareil permettant d'analyser visuellement la bandelette réactive.

6. Fixateur pour prolonger le temps d'évaluation représentatif d'une bandelette réactive (3), d'un immunodosage par compétition, garnie d'un échantillon provenant d'un organisme humain ou animal, destinée à l'utilisation dans un procédé selon l'une des revendications précédentes, composé essentiellement de cyclohexasiloxane, de diméthicone et de disiloxane, sachant qu'il contient au moins 55 % de cyclohexasiloxane, au moins 28 % de diméthicone et au moins 3 % de disiloxane.

7. Fixateur selon la revendication 6, **caractérisé en ce qu'**il contient au moins 60 %, de préférence 62 % de cyclohexasiloxane, au moins 30 %, de préférence 32 % de diméthicone et au moins 4 %, de préférence 5 % de disiloxane.

8. Set pour le contrôle et l'affichage d'absence ou de présence d'analytes dans un échantillon de liquides provenant de l'organisme humain ou animal, comprenant une plaque support (4) dotée d'au moins un réceptacle pour bandelette réactive dans lequel est disposée au moins une bandelette réactive (3) présentant un segment réceptacle (32) d'échantillon et un segment réactif (34), bandelette qui est dotée d'anticorps harmonisés avec ces analytes et conjugués à des particules de couleur, notamment des particules d'or, **caractérisé en ce qu'**est présent au moins un réservoir rempli d'un fixateur à base de silicone approprié pour conserver l'intensité des lignes d'au moins une bandelette réactive (3), réservoir qui est relié à un dispositif de dosage pour appliquer le fixateur sur au moins une bandelette réactive.

9. Set selon la revendication 8, **caractérisé en ce que** le réservoir est configuré en forme de pipette à usage unique reliée à la plaque support (4) de préférence via une jonction à séparation programmée, jonction qui présente de préférence une ligne de perforation.

10. Set selon la revendication 8, **caractérisé en ce qu'**est présente une enveloppe pour recevoir et recouvrir la plaque support (4), plaque contre laquelle le réservoir est fixé de sorte à se trouver disposé, après réception conforme de la plaque support (4), au-dessus du segment réceptacle (32) d'échantillon, sachant que de préférence sur la plaque support (4) se trouvent des moyens pour ouvrir le réservoir, positionnés de sorte que lors de la rentrée de la plaque support (4) dans l'enveloppe, ils longent le réservoir de sorte à ouvrir ce dernier, sachant que les moyens d'ouverture du réservoir sont de préférence configurés en forme d'élément de coupe ou de séparation.

11. Set selon la revendication 8, **caractérisé en ce que** ce dernier comprend un appareil (1) d'analyse visuelle du segment réactif (34) d'au moins une bandelette réactive (3), appareil qui présente un tiroir (15) recevant la plaque support (4) ainsi qu'un réservoir (22) rempli d'un fixateur, réservoir qui est relié à un dispositif de dosage (21), dispositif via lequel il est possible d'appliquer le fixateur venant du réservoir (22) sur le segment d'application (32) de l'échantillon d'au moins une bandelette réactive (3) de la plaque support (4) rentrée.

12. Appareil d'analyse visuelle d'au moins une bandelette réactive (3), d'un immunodosage par compétition, disposée sur une plaque support (4) et présentant un segment réceptacle (32) d'échantillon et un segment réactif (34), comprenant un tiroir (15) servant à recevoir la plaque support (4) ainsi qu'une unité d'éclairage pour illuminer uniformément la plaque support (4), au moins dans la zone du segment réactif (34) d'au moins une bandelette réactive (3), **caractérisé en ce qu'**est disposé un réservoir (22) rempli d'un fixateur à base de silicone approprié pour conserver l'intensité des lignes d'au moins une bandelette réactive (3), réservoir qui est relié à un dispositif de dosage (21) pour appliquer du fixateur sur le segment réceptacle (32) d'échantillon d'au moins une bandelette réactive (3) d'une plaque support (4) reçue par le tiroir (15).

13. Appareil selon la revendication 12, **caractérisé en ce que** le réservoir (22) est configuré comme une cartouche disposée détachable de l'appareil.

14. Cartouche à utiliser dans un appareil selon la revendication 12 ou 13, **caractérisée en ce qu'**elle est remplie d'un fixateur selon la revendication 6 ou 7.
